(19) Europäisches Patentamt
European Patent Office
Office européen des brevets

(11) Publication number: **0 496 691 A1**

# (12) EUROPEAN PATENT APPLICATION

(21) Application number: **92500007.7**

(22) Date of filing: **23.01.92**

(51) Int. Cl.[5]: **C07D 333/38,** C07D 333/28, C07D 333/24, C07D 333/20, C07D 295/22, C07D 295/18, C07D 295/12, C07D 213/82, C07D 207/34

(30) Priority: **24.01.91 ES 9100181**

(43) Date of publication of application: **29.07.92 Bulletin 92/31**

(84) Designated Contracting States: **AT BE CH DE DK FR GB GR IT LI LU NL SE**

(71) Applicant: **FABRICA ESPANOLA DE PRODUCTOS QUIMICOS Y FARMACEUTICOS, S.A. (FAES)**
**c/ Maximo Aguirre, 14**
**E-48940 Leioa-Lamiaco (Vizcaya) (ES)**

(72) Inventor: **Orjales Venero, Aurelio**
**P. del Puerto 24**
**Neguri Vizcaya) (ES)**
Inventor: **Alonso Cirées, Luisa**
**Plaza Xaho, 2-5 C**
**E-48940 Leioa (Vizcaya) (ES)**

(74) Representative: **Isern-Cuyas, Maria Luisa**
**Paseo de la Castellana 131, Bajo C**
**E-28046 Madrid (ES)**

(54) **Diphenylmethylpiperazine derivatives.**

(57) New diphenylmethylpiperazine derivatives are described with the following formula:

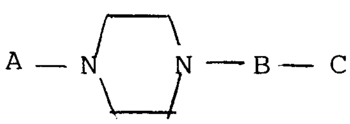

where A is a benzhydryl group, optionally substituted by a halogen atom, preferably chlorine; B is a carbonyl, acylamino, acylaminoalkyl or methylene group, and C is an aryl group, substituted or otherwise, a heteroaryl group, substituted or otherwise, an arylalkyl or heteroarylalkyl group, and their addition salts with pharmaceutically acceptable acids. these compounds have a pharmacologic, namely antihistaminic and anti-allergic, activity.

EP 0 496 691 A1

The present invention relates to a number of new diphenylmethylpiperazine derivatives of formula I and their addition salts with pharmaceutically acceptable acids.

The new diphenylmethylpiperazine derivatives have the following general formula

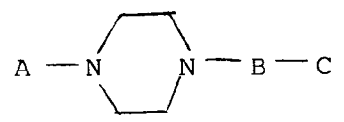

I

where A is a benzhydryl group, optionally substituted by a halogen, preferably chlorine; B is a carbonyl, acylamino, acylamino group N-substituted by a short chain alkyl group, or a short chain alkyl group, and C is an aryl, substituted or otherwise, heteroaryl, substituted or otherwise, arylalkyl or heteroarylalkyl group, the alkyl being of the short chain kind. The substituents of the aryl and heteroaryl groups are a halogen, preferably chlorine, and/or the methoxy, methyl and amino groups. Among the pharmaceutically acceptable acids for preparing addition salts are inorganic and organic acids such as hydrochloric, oxalic and fumaric acids.

The new diphenylmethylpiperazine derivatives subject of this patent are obtained by reacting an amine with the following general formula:

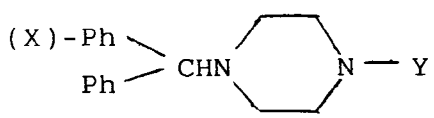

II

where X can be hydrogen or halogen, preferably chlorine, and Y can be hydrogen, an amino group, or an alkylenamino group wherein the carbonated chain contains two, three or four carbon atoms, with a benzoic acid chloride, which may be substituted in one or several positions by a halogen, an amino group, a methoxy group or a methyl group; or with heteroaromatic acid chloride, wherein the heteroatom can be oxygen, sulphur or nitrogen which forms part of a cycle of four or five carbon atoms and which may be substituted by one or more methyl groups, a halogen that can be chlorine, or an aromatic ring; or with an alkanoic or alkenoic acid chloride, substituted by a heteroaromatic cycle or by a phenyl group. The reaction takes place in a suitable organic solvent, such as dichloromethane, tetrahydrofurane or ether, and at room temperature, in the presence of basic substances such as triethylamine, pyridine or sodium bicarbonate.

The compounds wherein B, in general formula I, is a methylene group, are obtained by reducing the relevant compounds wherein B is a carbonyl group, with a suitable reducing agent, such as aluminium and lithium hydride, sodium and sodium borohydride.

The compounds subject of the present invention are pharmacologically active, as set out in the activity assays made. Thus, using the techniques originally described by Mota (Life Sciences, 12, 917, 1963) and Lefebvre et al. (C. R. Soc. Biol., 156, 183, 1962) it has been verified that all the compounds have antihistaminic and anti-allergic activity, most of them reaching a level within the range shown by the compounds use as reference, namely terphenadine and ketotiphene.

The following examples provide more details on the invention without limiting it to the same.

Example 1

Preparing 1-diphenylmethyl-4-(2-thiophencarbonyl)piperazine (1.)

2.5 g of benzhydrylpiperazine were dissolved in 20 ml of tetrahydrofurane, adding 1.5 ml of triethylamine. 1.5 g of thenoyl chloride dissolved in 20 ml of tetrahydrofurane were added dropwise at 0°C to the above solution. When addition was over, the solution was stirred at room temperature for one hour, whereupon 40 ml of water were added and 20 ml of dichloromethane were used for extraction, three times. The organic layer was separated and dried with anhydrous sodium sulphate, passing the solvent over in a vacuum. 3.7 g of a white solid were obtained, purified by column chromatography using dichloromethane/methanol (9/1) as eluent. M.P.: 127-8°C. The following were analogously prepared:

2. 1-(4-chlorobenzhydryl)-4-(2-thiophencarbonyl)piperazine. Breaks down on melting.
3. 1-diphenylmethyl-4(3-indolylcarbonyl)piperazine. M.P.: exceeds 260°C.
4. 1-(4-amino-5-chloro-2-methoxybenzoyl)-4-diphenylmethylpiperazine. M.P.: 190-2°C.

5. 1-(4-amino-5-chloro-2-methoxybenzoyl)-4-(4-chlorobenzhydryl)piperazine. M.P.: 152-4°C.

Example 2

Preparing N-(4-diphenylmethyl-1-piperazinyl)-2-thiophencarboxamide (6.)

2.7 g of 1-amino-4-diphenylmethylpiperazine were dissolved in 20 ml of dichloromethane, then adding 1.5 g of pyridine. 1.5 g of thenoyl chloride dissolved in 20 ml of dichloromethane were added dropwise at 0°C. Stirred for one hour, at room temperature, after which 40 ml of water were added and extraction took place, adding 20 ml of dichloromethane twice more. The organic layer was dried with anhydrous sodium sulphate and the solvent was eliminated by passing over in a vacuum. 3.8 g were obtained of a solid which was recrystallised in ethanol/water, with a melting point of 190-2°C.

The following were analogously prepared:

7. N-(4-diphenylmethyl-1-piperazinyl)-4-amino-5-chloro-2-methoxybenzamide. M.P.: 223-5°C.
8. N-(4-diphenylmethyl-1-piperazinyl)benzamide.M.P.:215-7°C.
9. N-(4-diphenylmethyl-1-piperazinyl)3-methylbenzamide. M.P.: 134-6°C.
10. N-(4-diphenylmethyl-1-piperazinyl)-3-phenylpropenamide. M.P.: 177-9°C.
11. N-(4-diphenylmethyl-1-piperazinyl)-3-pyridincarboxamide. M.P.: 188-90°C.
12. N-[4-(4-chlorobenzhydryl)-1-piperazinyl]-4-amino-5-chloro-2-methoxybenzamide. M.P.: 134-6°C.
13. N-[4-(4-chlorobenzhydryl)-1-piperazinyl]-2-thiophencarboxamide. M.P.: breaks down.
14. N-[4-(4-chlorobenzhydryl)-1-piperazinyl]-3-thiophencarboxamide. M.P.: 170-2°C.
15. N-[4-(4-chlorobenzhydryl)-1-piperazinyl]-5-methyl-2-thiophencarboxamide. M.P.: breaks down.

Example 3

Preparing N-[4-(4-diphenylmethyl-1-piperazinyl)butyl]-3-pyridincarboxamide (16.)

2.0 g of 1-(4-aminobutyl)-4-diphenylmethylpiperazine were dissolved in 20 ml of dichloromethane, subsequently adding 4.2 ml of triethylamine. 1.6 g of 3-pyridincarbonyl chloride dissolved in 20 ml of dichloromethane were added dropwise at 0°C to the above solution. After one hour, 40 ml of water were added, separating organic layer, which was dried with anhydrous sodium sulphate. After eliminating the solvents in a vacuum, 2.8 g of product remained which were purified by column chromatography using dichloromethane/methanol (9/1) as eluent. M.P.: 101-3°C.

The following were analogously prepared:

17. N-[2-(4-diphenylmethyl-1-piperazinyl)ethyl]-4-amino-5-chloro-2-methoxybenzamide. M.P.: 177-9°C.
18. N-[3-(4-diphenylmethyl-1-piperazinyl)propyl]-4-amino-5-chloro-2-methoxybenzamide. M.P.: 68-70°C.
19. N-[2-(4-diphenylmethyl-1-piperazinyl)ethyl]-2-thiophencarboxamide. M.P.: 149-51°C.
20. N-[2-(4-diphenylmethyl-1-piperazinyl)ethyl]-3-thiophencarboxamide. M.P.: breaks down.
21. N-[2-(4-diphenylmethyl-1-piperazinyl)ethyl]-3-methyl-2-thiophencarboxamide. M.P.: 113-5°C.
22. N-[2-(4-diphenylmethyl-1-piperazinyl)ethyl]-5-methyl-2-thiophencarboxamide. M.P.: 149-51°C.
23. N-[2-(4-diphenylmethyl-1-piperazinyl)ethyl]-3-chloro-2-thiophencarboxamide. M.P.: 98-100°C.
24. N-[2-(4-diphenylmethyl-1-piperazinyl)ethyl]-2-thiophenacetamide. M.P.: 130-2°C.
25. N-[2-(4-diphenylmethyl-1-piperazinyl)ethyl]-3-thiophenacetamide. M.P.: 125-7°C.
26. N-[2-(4-diphenylmethyl-1-piperazinyl)ethyl]-2-thiophenacrylamide. M.P.: breaks down.
27. N-[3-(4-diphenylmethyl-1-piperazinyl)propyl]-2-thiophencarboxamide. M.P.: 118-20°C.
28. N-[3-(4-diphenylmethyl-1-piperazinyl)propyl]-3-thiophencarboxamide. M.P.: 109-11°C.
29. N-[3-(4-diphenylmethyl-1-piperazinyl)propyl]-3-methyl-2-thiophencarboxamide. M.P.: 95-7°C.
30. N-[3-(4-diphenylmethyl-1-piperazinyl)propyl]-5-methyl-2-thiophencarboxamide. M.P.: breaks down.
31. N-[3-(4-diphenylmethyl-1-piperazinyl)propyl]-3-chloro-2-thiophencarboxamide. M.P.: breaks down.
32. N-[3-(4-diphenylmethyl-1-piperazinyl)propyl]-2-thiophenacetamide. M.P.: 98-100°C.
33. N-[3-(4-diphenylmethyl-1-piperazinyl)propyl]-3-thiophenacetamide. M.P.: breaks down.
34. N-[3-(4-diphenylmethyl-1-piperazinyl)propyl]-2-thiophenacrylamide. M.P.: breaks down.
35. N-[4-(4-diphenylmethyl-1-piperazinyl)butyl]-3-thiophencarboxamide. M.P.: 128-30°C.
36. N-[4-(4-diphenylmethyl-1-piperazinyl)butyl]-3-methyl-2-thiophencarboxamide. M.P.: 115-7°C.
37. N-[4-(4-diphenylmethyl-1-piperazinyl)butyl]-5-methyl-2-thiophencarboxamide. M.P.: 115-7°C.
38. N-[2-(4-diphenylmethyl-1-piperazinyl)ethyl]-2.4-dichlorobenzamide. M.P.: 127-9°C.
39. N-[2-(4-diphenylmethyl-1-piperazinyl)ethyl]-phenylacetamide. M.P.: 146-8°C.
40. N-[2-(4-diphenylmethyl-1-piperazinyl)ethyl]-3-phenylpropenamide. M.P.: 117-9°C.

41. N-[2-(4-diphenylmethyl-1-piperazinyl)ethyl]-3-pyridincarboxamide. M.P.: 140-2°C.
42. N-[2-(4-diphenylmethyl-1-piperazinyl)ethyl]-2-pyrrolcarboxamide. M.P.: 152-4°C.
43. N-[2-(4-diphenylmethyl-1-piperazinyl)ethyl]-3-indolcarboxamide. M.P.: breaks down.
44. N-[3-(4-diphenylmethyl-1-piperazinyl)propyl]-2.4-dichlorobenzamide. M.P.: 151-3°C.
45. N-[3-(4-diphenylmethyl-1-piperazinyl)propyl]-phenylacetamide. M.P.: breaks down.
46. N-[3-(4-diphenylmethyl-1-piperazinyl)propyl]-3-pyridincarboxamide. M.P.: 101-3°C.
47. N-[3-(4-diphenylmethyl-1-piperazinyl)propyl]-2-pyrrolcarboxamide. M.P.: 153-5°C.
48. N-[3-(4-diphenylmethyl-1-piperazinyl)propyl]-3-indolcarboxamide. M.P.: breaks down.
49. N-[4-(4-diphenylmethyl-1-piperazinyl)butyl]-2-pyrrolcarboxamide. M.P.: 118-20°C.
50. N-[4-(4-diphenylmethyl-1-piperazinyl)butyl]-3-indolcarboxamide. M.P.: 168-70°C.

Example 4

Preparing 1-(3-chloro-2-thiophenmethyl)-4-diphenylmethylpiperazine (51.)

0.5 g of aluminium and lithium hydride were suspended in 50 ml of dry ether. 3.0 g of 1-(3-chloro-2-thiophencarbonyl)-4-diphenylmethylpiperazine were added slowly, stirring for ten hours at room temperature. Hydrolised with 20 ml of water and 10 ml of NaOH at 10%. The alkaline hydroxides precipitated were separated by filtration and the filtrate extracted, with 20 ml of dichloromethane, three times. The organic layer was dried with anhydrous sodium sulphate and the solvent eliminated in a vacuum. 2.5 g of a product remained purified by column chromatography, using dichloromethane/methanol (9/1) as eluent. M.P.: 79-81°C.

The following was analogously prepared:

52. 1-diphenylmethyl-4-(3-thiophenmethyl)piperazine.M.P.:149-51°C.

## Claims

**1.** New diphenylmethylpiperazine derivatives, with the following general formula

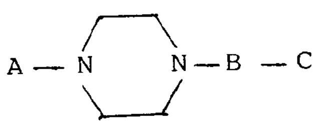

I

where A is a benzhydryl group, optionally substituted by a halogen, preferably chlorine, B is a carbonyl, acylamino, acylamino group N-substituted by a short chain alkyl group, or a short chain alkyl, and C is an aryl or heteroaryl group substituted or otherwise by a halogen, preferably chlorine, amino, methoxy or methyl, or an arylalkyl or heteroarylalkyl group, and their addition salts with pharmaceutically acceptable acids.

**2.** New diphenylmethylpiperazine derivatives, as in claim 1, characterised in being:

1. 1-diphenylmethyl-4-(2-thiophencarbonyl) piperazine.
2. 1-(4-chlorobenzhydryl)-4-(2-thiophencarbonyl) piperazine.
3. 1-diphenylmethyl-4(3-indolylcarbonyl)piperazine.
4. 1-(4-amino-5-chloro-2-methoxybenzoyl)-4-diphenylmethylpiperazine.
5. 1-(4-amino-5-chloro-2-methoxybenzoyl)-4-(4-chlorobenzhydryl)piperazine.
6. N-(4-diphenylmethyl-1-piperazinyl)-2-thiophencarboxamide.
7. N-(4-diphenylmethyl-1-piperazinyl)-4-amino-5-chloro-2-methoxybenzamide.
8. N-(4-diphenylmethyl-1-piperazinyl)benzamide.
9. N-(4-diphenylmethyl-1-piperazinyl)3-methylbenzamide.
10. N-(4-diphenylmethyl-1-piperazinyl)-3-phenylpropenamide.
11. N-(4-diphenylmethyl-1-piperazinyl)-3-pyridincarboxamide.
12. N-[4-(4-chlorobenzhydryl)-1-piperazinyl]-4-amino-5-chloro-2-methoxybenzamide.
13. N-[4-(4-chlorobenzhydryl)-1-piperazinyl]-2-thiophencarboxamide.
14. N-[4-(4-chlorobenzhydryl)-1-piperazinyl]-3-thiophencarboxamide.
15. N-[4-(4-chlorobenzhydryl)-1-piperazinyl]-5-methyl-2-thiophencarboxamide.
16. N-[4-(4-diphenylmethyl-1-piperazinyl)butyl]-3-pyridincarboxamide.

17. N-[2-(4-diphenylmethyl-1-piperazinyl)ethyl]-4-amino-5-chloro-2-methoxybenzamide.
18. N-[3-(4-diphenylmethyl-1-piperazinyl)propyl]-4-amino-5-chloro-2-methoxybenzamide.
19. N-[2-(4-diphenylmethyl-1-piperazinyl)ethyl]-2-thiophencarboxamide.
20. N-[2-(4-diphenylmethyl-1-piperazinyl)ethyl]-3-thiophencarboxamide.
21. N-[2-(4-diphenylmethyl-1-piperazinyl)ethyl]-3-methyl-2-thiophencarboxamide.
22. N-[2-(4-diphenylmethyl-1-piperazinyl)ethyl]-5-methyl-2-thiophencarboxamide.
23. N-[2-(4-diphenylmethyl-1-piperazinyl)ethyl]-3-chloro-2-thiophencarboxamide.
24. N-[2-(4-diphenylmethyl-1-piperazinyl)ethyl]-2-thiophenacetamide.
25. N-[2-(4-diphenylmethyl-1-piperazinyl)ethyl]-3-thiophenacetamide.
26. N-[2-(4-diphenylmethyl-1-piperazinyl)ethyl]-2-thiophenacrylamide.
27. N-[3-(4-diphenylmethyl-1-piperazinyl)propyl]-2-thiophencarboxamide.
28. N-[3-(4-diphenylmethyl-1-piperazinyl)propyl]-3-thiophencarboxamide.
29. N-[3-(4-diphenylmethyl-1-piperazinyl)propyl]-3-methyl-2-thiophencarboxamide.
30. N-[3-(4-diphenylmethyl-1-piperazinyl)propyl]-5-methyl-2-thiophencarboxamide.
31. N-[3-(4-diphenylmethyl-1-piperazinyl)propyl]-3-chloro-2-thiophencarboxamide.
32. N-[3-(4-diphenylmethyl-1-piperazinyl)propyl]-2-thiophenacetamide.
33. N-[3-(4-diphenylmethyl-1-piperazinyl)propyl]-3-thiophenacetamide.
34. N-[3-(4-diphenylmethyl-1-piperazinyl)propyl]-2-thiophenacrylamide.
35. N-[4-(4-diphenylmethyl-1-piperazinyl)butyl]-3-thiophencarboxamide.
36. N-[4-(4-diphenylmethyl-1-piperazinyl)butyl]-3-methyl-2-thiophencarboxamide.
37. N-[4-(4-diphenylmethyl-1-piperazinyl)butyl]-5-methyl-2-thiophencarboxamide.
38. N-[2-(4-diphenylmethyl-1-piperazinyl)ethyl]-2.4-dichlorobenzamide.
39. N-[2-(4-diphenylmethyl-1-piperazinyl)ethyl]-phenylacetamide.
40. N-[2-(4-diphenylmethyl-1-piperazinyl)ethyl]-3-phenylpropenamide.
41. N-[2-(4-diphenylmethyl-1-piperazinyl)ethyl]-3-pyridincarboxamide.
42. N-[2-(4-diphenylmethyl-1-piperazinyl)ethyl]-2-pyrrolcarboxamide.
43. N-[2-(4-diphenylmethyl-1-piperazinyl)ethyl]-3-indolcarboxamide.
44. N-[3-(4-diphenylmethyl-1-piperazinyl)propyl]-2.4-dichlorobenzamide.
45. N-[3-(4-diphenylmethyl-1-piperazinyl)propyl]-phenylacetamide.
46. N-[3-(4-diphenylmethyl-1-piperazinyl)propyl]-3-pyridincarboxamide.
47. N-[3-(4-diphenylmethyl-1-piperazinyl)propyl]-2-pyrrolcarboxamide.
48. N-[3-(4-diphenylmethyl-1-piperazinyl)propyl]-3-indolcarboxamide.
49. N-[4-(4-diphenylmethyl-1-piperazinyl)butyl]-2-pyrrolcarboxamide.
50. N-[4-(4-diphenylmethyl-1-piperazinyl)butyl]-3-indolcarboxamide.
51. 1-(3-chloro-2-thiophenmethyl)-4-diphenylmethylpiperazine.
52. 1-diphenylmethyl-4-(3-thiophenmethyl)piperazine.

**Claims for the following Contracting state: GR.**

1. A process for obtaining new diphenylmethylpiperazine derivatives with the following general formula

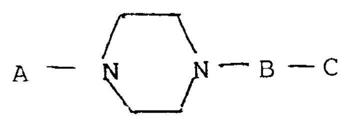

I

where A is a benzhydryl group, optionally substituted by a halogen, preferably chlorine, B is a carbonyl, acylamino, acylamino group N-substituted by a short chain alkyl group, or a short chain alkyl, and C is an aryl or heteroaryl group substituted or otherwise by a halogen, preferably chlorine, amino, methoxy or methyl, or an arylalkyl or heteroarylalkyl group, and their addition salts with pharmaceutically acceptable acids, characterised in reacting an N-substituted piperazine with an acid chloride in a suitable organic solvent such as dichloromethane, tetrahydrofurane and ether, in the presence of a base such as triethylamine, pyridine and sodium bicarbonate, for an hour, at room temperature.

2. A process for obtaining new dyphenylmethylpiperazine derivatives as in claim 1, characterised in that the N-substituted piperazine has the following formula

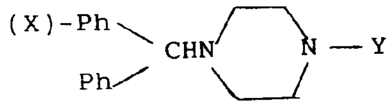

II

where X can be hydrogen or halogen, preferably chlorine, and Y can be a hydrogen, amino or alkylenamino group wherein the carbonated chain contains two, three or four carbon atoms.

3. A process for obtaining new diphenylmethylpiperazine derivatives, as in previous claims, characterised in that the acid chloride is a benzoic acid chloride, that may be substituted in one or more positions, by halogen, preferably chlorine, an amino group, a methoxy group or a methyl group, or a heteroaromatic acid wherein the heteroatom can be oxygen, sulphur and nitrogen, forming part of a cycle with four or five carbon atoms, which may be substituted by one more methyl groups, a halogen or an aromatic ring, or an alkanoic or alkenoic acid substituted by a heteroaromatic cycle or a phenyl group.

4. A process for obtaining new diphenylmethylpiperazine derivatives, as in previous claims, characterised in that the compounds wherein B, in the general formula I, is a short chain alkyl, preferably methylene , obtained by reduction, with a suitably reducing agent such as aluminium and lithium hydride, from the relevant compounds in which B is a carbonyl group.

European Patent Office

# PARTIAL EUROPEAN SEARCH REPORT

which under Rule 45 of the European Patent Convention shall be considered, for the purposes of subsequent proceedings, as the European search report

Application Number
EP 92 50 0007

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,A | EP-A-0 434 561 (ADIR ET COMPAGNIE)<br>* Abstract; claims 1,6,9 * | 1,3 | C 07 D 333/38<br>C 07 D 333/28<br>C 07 D 333/24<br>C 07 D 333/20<br>C 07 D 295/22<br>C 07 D 295/18<br>C 07 D 295/12<br>C 07 D 213/82<br>C 07 D 207/34 |
| X | EP-A-0 210 782 (DAINIPPON PHARMACEUTICAL CO., LTD)<br>* Abstract; claims 1-3,10,11; tables 5,7 * | 1,3,4 | |
| X | EP-A-0 113 226 (FUJISAWA PHARMACEUTICAL CO., LTD)<br>* Abstract; claims 1,8 * | 1,3,4 | |
| X | CHEMICAL AND PHARMACEUTICAL BULLETIN, vol. 37, no. 1, 1989, pages 100-105, Toyko, JP; Y. NISHIKAWA et al.: "Acrylamide derivatives as antiallergic agents. I. Synthesis and structure-activity relationships of N-[(4-substituted 1-piperazinyl)alkyl]-3-(aryl and heteroaryl)acrylamides"<br>* Whole document *<br>-/- | 1 | TECHNICAL FIELDS SEARCHED (Int. Cl.5)<br>C 07 D |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely : 1-6

Claims not searched :

Reason for the limitation of the search:

As the drafting of the claims is not clear and concise (Art. 83-84 EPC) and encompasses such an enormous amount of products, a complete search is not possible on economic grounds (see Guidelines for examination in the EPO, Part B, Chapter III, 2). So the search has been limited (Rule 45) to the inventive concept as illustrated in the examples.

| Place of search | Date of completion of the search | Examiner |
|---|---|---|
| THE HAGUE | 13-04-1992 | PAISDOR B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

Page 2

European Patent Office

# PARTIAL EUROPEAN SEARCH REPORT

Application Number

EP 92 50 0007

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF MEDICINAL CHEMISTRY, vol. 32, no. 3, 1989, pages 583-593, Washington, US; Y. NISHIKAWA et al.: "Acrylamide derivatives as antiallergic agents. 2. Synthesis and structure-activity relationships of N-[4-(diphenylmethyl)-1-piperazinyl]butyl]-3-(3-pyridyl)acrylamides" * Whole document * --- | 1 | |
| X | CHEMICAL AND PHARMACEUTICAL BULLETIN, vol. 37, no. 3, 1989, pages 684-687, Toyko, JP; Y. NISHIKAWA et al.: "Acrylamide derivatives as antiallergic agents. III. Synthesis and structure-activity relationships of N-[4-(4-diphenylmethyl-1-piperazinyl)butyl]- and N-[4-(4-diphenylmethylene-1-piperidyl)butyl]-3-heteroarylacrylamides" * Whole document * --- | 1,3 | TECHNICAL FIELDS SEARCHED (Int. Cl.5) |
| X | CHEMICAL ABSTRACTS, vol. 82, no. 23, 9th June 1975, page 618, column 1, abstract no. 156367d, Columbus, Ohio, US; & JP-A-49 093 379 (YOSHITOMI PHARMACEUTICAL INDUSTRIES LTD) 05-09-1974 * Abstract * ----- | 1 | |

EPO FORM 1503 03.82 (P0410)